# EUROPEAN PATENT SPECIFICATION

(11) **EP 1 257 322 B1**
(45) Date of publication and mention of the grant of the patent: **16.11.2005**
(21) Application number: 01904680.4
(22) Date of filing: 23.01.2001
(51) Int. Cl.: A61N 1/372

(54) **WIRELESS COMMUNICATION SYSTEM FOR IMPLANTABLE MEDICAL DEVICES**
DRAHTLOSES KOMMUNIKATIONSSYSTEM FÜR IMPLANTIERBARE MEDIZINISCHE GERÄTE
SYSTEME DE COMMUNICATION SANS FIL POUR DISPOSITIF MEDICAUX IMPLANTABLES

(30) Priority: 07.02.2000 SE 0000372
(43) Date of publication of application: 20.11.2002
(73) Proprietor: St. Jude Medical AB, 175 84 Järfälla (SE)
(72) Inventor: ABRAHAMSON, Hans, S-113 38 Stockholm (SE)
(74) Representative: Kalling, Sven Owe
(86) International application number: PCT/SE2001/000123
(87) International publication number: WO 2001/056653

(56) References cited:
- US-A- 4 041 954
- US-A- 4 677 982
- US-A- 5 387 259
- US-A- 5 476 488
- US-A- 5 713 939
- O.Y. DE VEL: 'Controlled transcutaneous powering of a chronically inplanted telemetry device' BIOTELEMETRY PATIENT MONITG 6 1979, pages 176 - 185, XP002939813

## Description

### Technical field of the invention

The invention relates to a medical communication system, an extracorporeal communication module and an implantable communication module according to the preamble of the independent claim.

### Background of the invention

Communication between an implanted medical device, such as pacemakers, defibrillators and drug delivery systems, and an extracorporeal communication device is often performed, according to long established technique, with bi-directional radio wave telemetry using electromagnetic carrier waves for signaling in the radio frequency range (from a couple of Hz to some few MHz). The base band frequencies limit the data rate to a few or a few tens of kilobits per second. Radio wave communication used in connection with medical implants is prone under certain conditions to be disturbed by internal noise, e.g. shock capacitor charging in an implantable defibrillator, and external noise, e.g. CRT monitors and mobile telephones.

US-5,476,488 relates to a transmitter power control for implantable medical devices where an external programmer measures the strength of the telemetry signals transmitted by the implantable device. A power control signal is generated as a function of the measured signal strength and the control signal is transmitted to the implanted device that adjusts the transmitter power to a power level specified by the power control signal.

By instead using light to interchange information between an implanted device and an extracorporeal unit a communication link with higher transmission rates and which is insensitive to the above-mentioned disturbances related to radio wave telemetry is achieved.

US-4,677,982 discloses an apparatus for transcutaneous communication using infrared light signals to communicate bi-directionally between electronic apparatus implanted within a living organism and electronic apparatus external to the body. The internal circuitry consumes power from an internal battery only when the external apparatus requests implant activation.

An overall concern when designing and specifying medical devices intended for long-time implantation is the power consumption of the device. One important object of a communication module of a medical implant is therefore to minimize or even eliminate the drain of the battery of the implanted device when communicating with the device.

A data communication system for control of transcutaneous energy transmission to an implantable medical device is disclosed in US-5,713,939 having an implantable medical device with rechargeable batteries and a single coil that is employed both for energy transmission and data telemetry. The energy transfer is controlled based upon signals generated in the implantable device that indicate the battery's state of charge or discharge.

US-4,041,954 discloses a system for detecting information in an artificial cardiac pacemaker by using energy supplied from the outside of the pacemaker. According to the US-patent is it undesirable to use the battery energy in the pacemaker for transmitting information signals from the pacemaker because such shortens the life of the battery and thus the life of the pacemaker itself. Energy is supplied from the outside to the energy receiving means in the implanted pacemaker. This received energy is used to generate an information energy signal that is transmitted to the outside of the pacemaker. The information energy signal is created from signals representing e.g. the power consumption of the battery in the pacemaker. The signals transmitted to and from the implanted pacemaker are e.g. light signals.

In US-5,387,259 is disclosed an optical transdermal link between an internal module placed just inside the skin and an external module placed just outside the skin and facing the internal module. The internal module contains a photocell array to provide power, received from the external module, for itself and other devices. The optical link is also used to bi-directionally transmit data between the modules. The internal module also contains neural interface circuits peculiar to a specific application.

Biotelemetry Patient Monitg 6, pp.176-185, (1979) O.Y. De Vel: "Controlled Trancutaneous Powering of a Chronically Implanted Telemetry Device" discloses a transcutaneous energy transmission system control. The "Biotelemetry" system control is undertaken by two subunits in the external power transfer module:(1) an automatic frequency control (AFC) and (2) an automatic energy control unit (AEC). AFC selects an optimal frequency when maximum primary current (maximum power emission) is detected. No communication signal from the implanted module is involved. AEC determines the duration of the energy emission cycle from the external module and the energy output is terminated in response to the detection in the external module of a 2 MHz communication signal received from the implanted module. The 2 MHz signal is sent out in reponse to that a predetermined voltage level in the implant has been reached.

To summarize, the "Biotelemetry" as well as the other prior art systems mentioned above may send out a communication signal from the implant to be received by the external power transfer module, where the communication signal contains information related to a parameter (such as the battery's state of charge) in the implant, and where that information is used to control the power transmission from the external power transfer module. However, the prior art does not disclose that a measure of the quality of the communication signal as such received at an external module should be used to control the power transmission to an internal communication module from the external module comprising energy generating and transferring means in accordance with the subject-matter of the present invention as explained below.

The object of the present invention is to achieve an improved transcutaneous medical communication system having a high transmission rate, that is insensitive to disturbances and that eliminates using the implanted device's battery.

### Summary of the invention.

The above-mentioned object is achieved in that a medical communication system, an external communication module and an internal communication module according to the preambles of the appended independent claims are provided with the features set forth in characterizing parts of the independent claims.

The medical communication system according to the invention achieves optimized energy transmission from an external communication module to the internal communication module in dependence of a pre-determined measure of the quality of the information data transmitted from the internal module.

Preferred embodiments of the invention are set forth in the dependent claims.

According to a preferred embodiment of the invention the communication signal is an optical signal transmitted according to the Manchester coding algorithm.

According to still another preferred embodiment acceptable communication quality of the communication signal is achieved if the determined measure of quality of the communication signal lies in an interval between a first value and a second value. The lower boundary defined by the first value is set to a level ensuring that enough energy is transmitted to the implanted module in order to achieve acceptable communication quality. The higher boundary defined by the second value is set to a level ensuring that not too much energy is transmitted to the implanted module thus avoiding that the circuits in the implanted device unnecessarily are exposed to higher currents.

### Short description of the appended drawings

Figure 1 shows a schematic block diagram of the present invention;
Figure 2 illustrates the principles of the Manchester coding, and
Figure 3 shows a detailed block diagram according to a preferred embodiment of the invention.

### Detailed description of preferred embodiments of the invention.

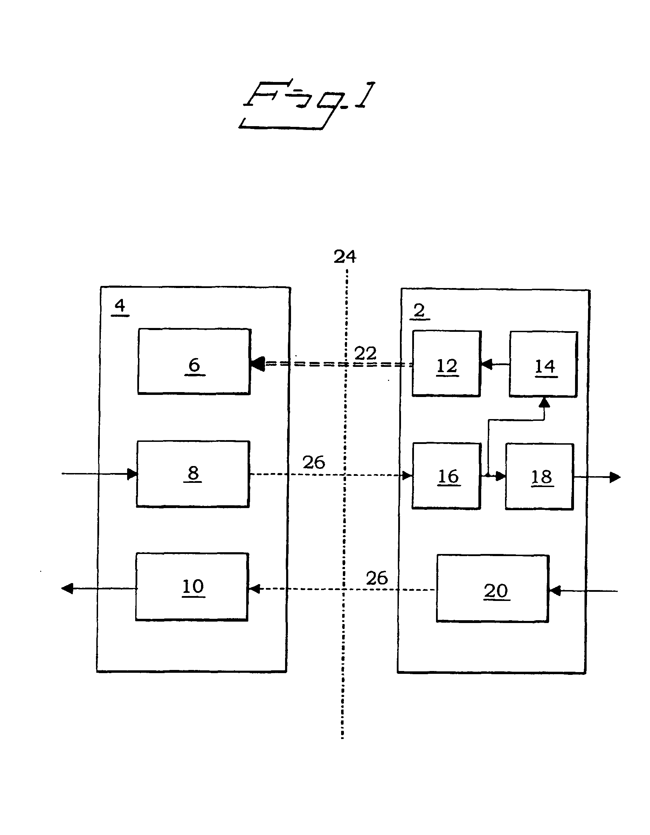

Figure 1 shows a schematic block diagram disclosing a medical communication system according to the present invention. The system comprises an external communication module 2 arranged extracorporeally of a patient and an implantable internal communication module 4. The internal communication module 4 is adapted to be arranged to be connectable to or comprised in an implantable medical device (not shown), e.g. a pacemaker, defibrillator, cardioverter or drug delivery pump system. The internal communication module 4 comprises energy receiving and supplying means 6 adapted to supply the communication module 4 with energy, internal signal transmitting means 8 and internal signal receiving means 10.

The external communication module 2 is adapted to be arranged to be connectable to or comprised in an external programming device (not shown), e.g. a so called programmer, suitable for controlling the performance of the implant. The external communication module 2 comprises energy generating and transferring means 12, signal analyzing means 14, signal receiving means 16, signal decoding means 18 and signal transmitting means 20.

The energy receiving and supplying means 6 in the internal communication module 4 can wirelessly receive energy 22 through the skin 24 of the patient from the energy generating and transmitting means 12 in the external communication module 2 by any wireless means, e.g. infrared light or RF-waves.

In medical implants of today it is often possible to store information related to the therapy performed by the implant, e.g. the pacing mode, interval lengths, detected physical parameters or the stimulation pulse amplitude in a pacemaker. This type of information is transmitted via telemetry from the implant to the physician for evaluation. The physician can change certain parameters of the implant related to the therapy or request further information regarding specific issues. These commands or data requests are transmitted via telemetry to the implant from an external unit.

The information data to be sent out from the implant is applied, according to the present invention, to the internal signal transmitting means 8 where it is encoded according to a preferred coding algorithm which will be described in detail below. The encoded information data is transmitted as a wireless communication signal 26 through the skin to the signal receiving means 16 in the external communication module 2. The thus received signal is applied to the signal analyzing means 14 and to the signal decoding means 18 where the signal is decoded according to the coding algorithm and the original information data is made available via the external programming device (not shown).
The signal analyzing means 14 analyzes the received signal and determines if a measure of the quality of the signal fulfils a predetermined criterion. The criterion comprises an interval between a first value and a second value and acceptable communication quality is achieved if said measure of the quality lies in that interval. In response of the analysis performed by the signal analyzing means the energy generating and transferring means 12 is ordered to change the amount of energy transferred to the energy receiving and supplying means 6 according to energy transferring rules. According to these rules is the amount of transferred energy increased if the measure of the quality is below said first value and the amount of transferred energy is decreased if the measure of quality is above said second value.

The information data to be sent into the implant is applied to the signal transmitting means 20 of the external communication module 2 where it is encoded according to a preferred coding algorithm. The encoded information is then transferred as a communication signal 26 to the internal signal receiving means 10 in the internal communication module 4 where the received communication signal is decoded according to the coding algorithm. The thus decoded information is then applied to the medical device (not shown). The communication signal between the external and internal modules could be an optical signal as described above in the referenced prior art. The communication signal could also be a radio wave signal, which is a commonly used communication technique used for communicating with a medical implant, e.g. a pacemaker, or any other suitable signal, e.g. ultra-sound.

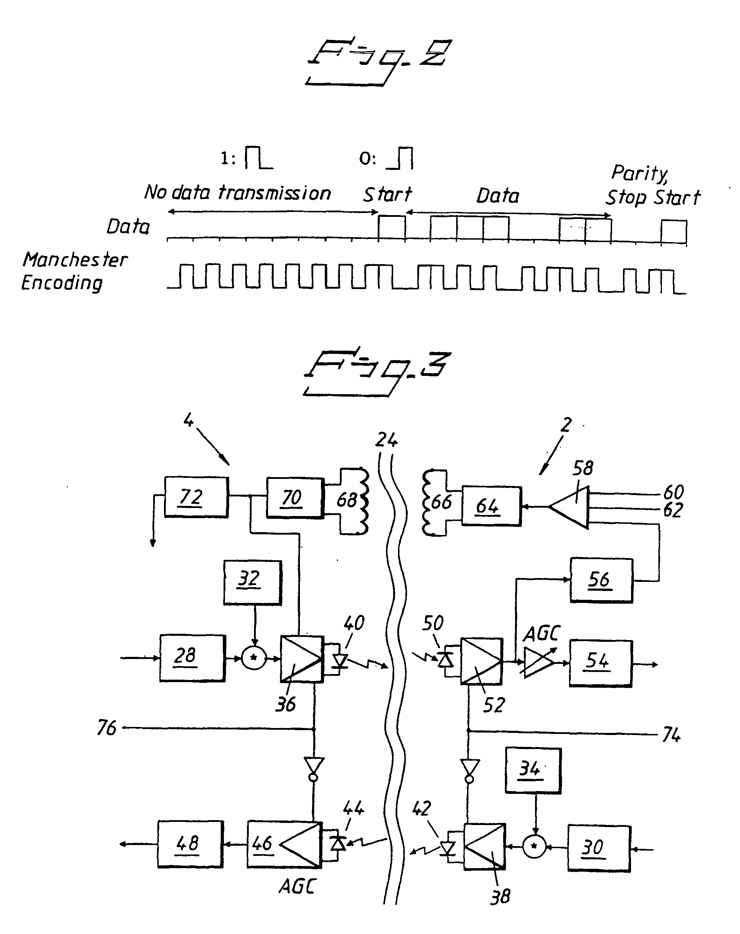

Figure 2 illustrates the principles of the Manchester coding which is a preferred coding algorithm used in the present invention.
It should be noted that any coding algorithm could be used provided that, even if no information is transferred or only "zeros" is transferred, a waveform still is transferred. The reason for that is to make it possible to monitor the quality of the communication signal.
As can be seen on the top of figure 2 is the information carrying symbol logic 1 encoded as a square pulse having a "high" first half and a "low" second half. Information carrying symbol logic 0 is encoded as a square pulse having a "low" first half and a "high" second half.
The information data to be transmitted is seen as a number of bits in the row designated "Data". Although this is only a simple example to illustrate the principle of the Manchester coding it also illustrates an other important aspect of communication, namely the communication protocol used, i.e. how a package of information data is surrounded by a start bit, a parity bit and a stop bit. In this example eight information data bits are surrounded by a start bit that always is logic 1 and ended by a parity bit (its value depends on the information data) and a stop bit that is always logic 0. The Manchester coding of this data stream is seen below in the figure.
In the disclosed coding algorithm is the representation of the logic 1 and logic 0 of equal length, but it is of course also possible to use different lengths. For example, a 40/60 representation means that the information carrying symbol logic 1 is encoded as a square pulse being "high" the first 40% and "low" the rest of the whole interval. A logic 0 is then encoded as a square pulse being "low" the first 60% and "high" the rest of the whole interval.

In the coding algorithm disclosed above, e.g. the Manchester coding algorithm, a waveform is transferred irrespectively of whether a logic 0 or a logic 1 is transferred in order to monitor the quality of the communication signal. According to an alternative embodiment of the invention is instead a conventional on-off-keying used, i.e. a logic 0 is transmitted as "low" and logic 1 is transmitted as "high" during the whole interval, respectively. In order to determine a measure of quality of a message thus encoded, a known checking sequence of ones and zeros is interleaved in the message at known time intervals. The received signal is only analyzed and a measure of quality is only determined when the checking sequence is received. Thus, the signal analyzing means 14 is only activated in response to a received checking sequence.

In figure 3 a detailed block diagram is shown of a preferred embodiment of the invention. According to this preferred embodiment is the communication between the external and internal modules performed by an optical signal and preferably by an infra-red (IR) signal.
The signal transmitting means 8,20 in the external and internal modules, respectively, both comprise an encoder 28,30, preferably according to the Manchester coding algorithm, a carrier wave oscillator 32,34, an LED driver 36,38 and a light emitting diode (LED) 40,42.
The information data to be sent in either direction are first encoded by the encoder 28,30 and then modulated by a high frequency carrier wave before the signal is applied to the LED driver 36,38. In the modulated encoded signal is the waveform that corresponds to logic 0 phase shifted 180° compared to the waveform that corresponds to logic 1.
The internal signal receiving means 10 comprises an IR-photo diode 44, signal processing means 46 that filters, demodulates and amplifies the detected signal. The filtration removes baseline undulations and disturbances due to background light and the demodulator retrieves the original waveform. An automatic gain control (AGC) is used to adjust the output from the IR-photo diode to an appropriate level to a succeeding decoder 48, preferably a Manchester decoder. The decoded information data is then supplied to the medical device (not shown).
The external signal receiving means 16 in the external communication module 2 comprises an IR-photo diode 50 and an external signal processing means 52 that filters, demodulates and amplifies the detected signal. The signal is then split in two paths.
The signal in one path is applied to an automatic gain control (AGC) to adjust the output from the IR-photo diode to an appropriate level to a succeeding decoder 54, preferably a Manchester decoder. The decoded information data is then supplied to a device (not shown), e.g. a programmer or part of a programmer, for evaluation of the information.
The signal in the other path is applied to the signal analyzing means 14 comprising a signal processor 56, e.g. an AM decoder/integrator, generating a signal proportional to the average amplitude of the signal received from the external signal processing means 52, and a level detector 58.
According to a preferred embodiment of the invention is a signal generated by the signal processor 56 instead proportional to the peak amplitude value or the mean of the peak amplitude value of the signal received from the external signal processing means 52.

As indicated above in connection with the description of figure 2 is a measure of the quality of the received signal determined by the signal processor 56. This measure is applied to the level detector 58, which is provided with a first value 60 and a second value 62 defining an interval where acceptable communication is achieved if said measure of the quality lies in that interval.
A quality criterion is defined by the interval between the first value and the second value.
The output signal from the level detector is applied to the energy generating means 12 comprising a logic and driver circuit 64 and an energy generating coil 66.

In response of the analysis performed by the signal analysing means as indicated above the energy generating and transferring means 12 is ordered to change the amount of energy transferred to the energy receiving and supplying means 6 according to energy transferring rules. According to these rules the amount of transferred energy is increased if the measure of the quality is below said first value and the amount of transferred energy is decreased if the measure of the quality is above said second value. The transferred energy is received by the energy receiving and supplying means 6 in the internal communication module 4. The energy supplying means comprises a coil 68, a rectifier 70 and a power regulator 72. The received energy is rectified by the rectifier 70 and applied to the power regulator 72 and to the LED driver 36. The output voltage from the rectifier 70 increases proportionally to the measure of the quality of the communication signal. In case of a low voltage, i.e. faint light, the result will be an increase in the amount of transferred energy resulting in an increase in the output energy from the rectifier 70 applied to the LED driver 36 and thus stronger emitted light from the LED 40. This will continue until the voltage reaches the second value and the transferred energy is then adjusted to keep the voltage within the interval between the first and second values.
The power regulator 72 provides the other functional units of the internal communication module with a constant, regulated voltage during communication transmission.

According to a preferred embodiment of the invention is the external and internal communication modules, respectively, provided with a transmitting/receiving control signals 74,76 in order to be able to control and synchronize the external and internal modules. The reason for that is that the internal and external modules cannot transmit at the same time, since the IR photo diodes would then risk to detect light from both their own LED and from the other module's LED. The two modules therefore have to work in half-duplex, i.e. only one direction can be active at a time. The control signals 74,76 turn the signal receiving means 10,16 off when the signal transmitting means 8,20 are transmitting. The internal module is the master that controls who is in turn to transmit a communication signal. If no communication data is transmitted, the internal module sends "null" data in order to keep up the power transmission feedback loop.

The present invention is not limited to the above-described preferred embodiments. Various alternatives, modifications and equivalents may be used. Therefore, the above embodiments should not be taken as limiting the scope of the invention, which is defined by the appendant claims.

## Claims

1. Medical communication system comprising an extracorporeal external communication module (2) and an implantable internal communication module (4) adapted to perform communication between each other, said internal communication module comprising energy receiving and supplying means (6) arranged to supply energy to said internal communication module and signal transmission means (8) separate from said energy receiving and supplying means, wherein said energy receiving and supplying means wirelessly can receive energy (22) from an energy generating and transferring means (12) in said external communication module, wherein said external communication module (2) comprises energy generating and transferring means (12) and, separate there from, further comprises a signal analysing means (14) that analyses a communication signal (26) received from the internal communication module (4) and determines a measure of the quality of the received communication signal as such and if said measure does not fulfil a predetermined criterion related to the communication signal the amount of energy transferred from the energy generating and transferring means (12) to the energy receiving and supplying means (6) is changed according to predetermined energy transferring rules.

2. Medical communication system according to claim 1, **characterized in that** said communication signal comprises information carrying symbols encoded according to a coding algorithm such that all symbols to be transmitted correspond to a transmitted waveform.

3. Medical communication system according to claim 2, **characterized in that** said coding algorithm used is the Manchester coding algorithm.

4. Medical communication system according to any preceding claim, **characterized in that** the energy transferred from the energy generating and transferring means to the energy receiving and supplying means is electrical energy supplied via induction.

5. Medical communication system according to any preceding claim, **characterized in that** said received communication signal is an optical signal and **in that** said measure of the received signal is proportional to the average amplitude of the received optical signal.

6. Medical communication system according to any of claims 1-4, **characterized in that** said measure of the received communication signal is proportional to the peak value or the mean of the peak value of the received communication signal.

7. Medical communication system according to any preceding claim, **characterized in that** said communication signal is optical.

8. Medical communication system according to any preceding claim, **characterized in said** communication is bi-directional.

9. Medical communication system according to any preceding claim, **characterized in that** said predetermined criterion comprises an interval between a first value and a second value, wherein acceptable communication quality is achieved if said measure of the quality lies **in that** interval.

10. Medical communication system according to claim 9, **characterized in that** if said measure is below said first value the amount of transferred energy is increased and if said measure is above said second value the amount of transferred energy is decreased by said energy generating means according to the predetermined energy transferring rules.

## Patentansprüche

1. Medizinisches Kommunikationssystem, enthaltend ein extracorporales, externes Kommunikationsmodul (2) und ein implantierbares, internes Kommunikationsmodul (4), die ausgelegt sind, miteinander zu kommunizieren, wobei das genannte interne Kommunikationsmodul eine Energieempfangs- und Versorgungsvorrichtung (6) enthält, die so ausgebildet ist, dass sie dem genannten internen Kommunikationsmodul und einer Signalsendevorrichtung (8) aus der genannten Energieempfangs- und Versorgungsvorrichtung getrennt Energie zuführen kann, wobei die genannte Energieempfangs- und Versorgungsvorrichtung drahtlos Energie (22) aus einer Energieerzeugungs- und Übertragungsvorrichtung (12) in dem genannten externen Kommunikationsmodul empfangen kann, wobei das genannte externe Kommunikationsmodul (2) eine Energieerzeugungs- und Übertragungsvorrichtung (12) enthält, und getrennt hiervon ferner eine Signalanalysiervorrichtung (14) enthält, die ein vom internen Kommunikationsmodul (4) empfangenes Kommunikationssignal (26) analysiert und ein Qualitätsmaß des empfangenen Kommunikationssignals als solches bestimmt und falls das genannte Maß ein sich auf das Kommunikationssignal bezogenes vorbestimmtes Kriterium nicht erfüllt, die von der Energieerzeugungs- und Übertragungsvorrichtung (12) zur Energieempfangs- und Versorgungsvorrichtung (6) übertragene Energiemenge entsprechend vorbestimmter Energieübertragungsregeln geändert wird.

2. Medizinisches Kommunikationssystem nach Anspruch 1, **dadurch gekennzeichnet, dass** das genannte Kommunikationssignal Informationen tragende Symbole enthält, die gemäß einem Kodieralgorithmus derart kodiert sind, dass sämtliche zu übertragenden Symbole einer übertragenen Wellenform entsprechen.

3. Medizinisches Kommunikationssystem nach Anspruch 2, **dadurch gekennzeichnet, dass** der genannte, benutzte Kodieralgorithmus der Manchester-Kodieralgorithmus ist.

4. Medizinisches Kommunikationssystem nach einem der vorhergehenden Ansprüche, **dadurch gekennzeichnet, dass** die aus der Energieerzeugungs- und Übertragungsvorrichtung zur Energieempfangs- und Versorgungsvorrichtung übertragene Energie eine mittels Induktion zugeführte elektrische Energie ist.

5. Medizinisches Kommunikationssystem nach einem der vorhergehenden Ansprüche, **dadurch gekennzeichnet, dass** das genannte, empfangene Kommunikationssignal ein optisches Signal ist, und dass das genannte Maß des empfangenen Signals proportional zur Durchschnittsamplitude des empfangenen optischen Signals ist.

6. Medizinisches Kommunikationssystem nach einem der Ansprüche 1 bis 4, **dadurch gekennzeichnet, dass** das genannte Maß des empfangenen Kommunikationssignals proportional zum Spitzenwert oder zum Mittel des Spitzenwertes des empfangenen Kommunikationssignals ist.

7. Medizinisches Kommunikationssystem nach einem der vorhergehenden Ansprüche, **dadurch gekennzeichnet, dass** das genannte Kommunikationssignal optisch ist.

8. Medizinisches Kommunikationssystem nach einem der vorhergehenden Ansprüche, **dadurch gekennzeichnet, dass** die genannte Kommunikation bidirektional erfolgt.

9. Medizinisches Kommunikationssystem nach einem der vorhergehenden Ansprüche, **dadurch gekennzeichnet, dass** das genannte vorbestimmte Kriterium ein Intervall zwischen einem ersten Wert und einem zweiten Wert umfasst, wobei eine akzeptierbare Kommunikationsqualität erzielt wird, falls das genannte Qualitätsmaß in dem Intervall liegt.

10. Medizinisches Kommunikationssystem nach Anspruch 9, **dadurch gekennzeichnet, dass** durch die genannte Energieerzeugungsvorrichtung, gemäß den vorbestimmten Energieübertragungsregeln, die Menge der übertragenen Energie erhöht wird, falls das genannte Maß unterhalb des genannten ersten Wertes liegt, und die Menge der übertragenen Energie verringert wird, falls das genannte Maß oberhalb des genannten zweiten Wertes liegt.

## Revendications

1. Système de communication médicale comprenant un module (2) de communication externe extracorporel et un module (4) de communication interne implantable conçus pour communiquer entre eux, le module de communication interne comprenant des moyens (6) de réception et d'alimentation en énergie destinés à alimenter en énergie le module de communication interne et des moyens (8) de transmission du signal distincts des moyens de réception et d'alimentation en énergie, les moyens de réception et d'alimentation en énergie pouvant recevoir sans fil de l'énergie (22) de moyens (12) de production et de transfert d'énergie du module de communication externe, dans lequel le module (2) de communication externe comprend des moyens (12) de production et de transfert d'énergie et séparément de ceux-ci comprend, en outre, des moyens (14) d'analyse du signal, qui analysent un signal (26) de communication reçu du module (4) de communication interne et déterminent une mesure de la qualité du signal de communication reçu en tant que tel et, si cette mesure ne satisfait pas un critère déterminé à l'avance lié au signal de communication, la quantité d'énergie transférée des moyens (12) de production et de transfert d'énergie aux moyens (6) de réception et d'alimentation en énergie est modifiée suivant des règles déterminées à l'avance de transfert d'énergie.

2. Système de communication médicale suivant la revendication 1, **caractérisé en ce que** le signal de communication comprend des symboles portant des informations codées suivant un algorithme de codage tel que tous les symboles à transmettre correspondent à une forme d'onde transmise.

3. Système de communication médicale suivant la revendication 2, **caractérisé en ce que** l'algorithme de codage utilisé est l'algorithme de codage Manchester.

4. Système de communication médicale suivant l'une quelconque des revendications précédentes, **caractérisé en ce que** l'énergie transférée des moyens de production et de transfert d'énergie aux moyens de réception et d'alimentation en énergie est de l'énergie électrique fournie par induction.

5. Système de communication médicale suivant l'une quelconque des revendications précédentes, **caractérisé en ce que** le signal de communication reçu est un signal optique et **en ce que** la mesure du signal reçu est proportionnelle à l'amplitude moyenne du signal optique reçu.

6. Système de communication médicale suivant l'une des revendications 1 à 4, **caractérisé en ce que** la mesure du signal de communication reçu est proportionnelle à la valeur de crête ou à la moyenne des valeurs de crête du signal de communication reçu.

7. Système de communication médicale suivant l'une quelconque des revendications précédentes, en ce que le signal de communication est optique.

8. Système de communication médicale suivant l'une quelconque des revendications précédentes, **caractérisé en ce que** la communication est bidirectionnelle.

9. Système de communication médicale suivant l'une quelconque des revendications précédentes, **caractérisé en ce que** le critère déterminé à l'avance comprend un intervalle entre une première valeur et une deuxième valeur, une qualité de communication acceptable étant obtenue si cette mesure de la qualité se trouve dans cet intervalle.

10. Système de communication médicale suivant la revendication 9, **caractérisé en ce que**, si la mesure est en dessous de la première valeur, la quantité d'énergie transférée est augmentée et, si la mesure est au-dessus de la deuxième valeur, la quantité d'énergie transférée est diminuée par les moyens de production d'énergie suivant les règles de transfert d'énergie déterminées à l'avance.
